# EUROPEAN PATENT APPLICATION

(11) **EP 1 667 050 A1**
(43) Date of publication of application: **07.06.2006**
(21) Application number: 04735484.0
(22) Date of filing: 31.05.2004
(51) Int. Cl.: G06K 11/00, G01L 1/00, A43B 17/04, A61G 7/057

(54) **INTELLIGENT LAMINATED PRESSURE SURFACE**

(30) Priority: 05.06.2003 ES 200301387
(71) Applicant: Chasco Pérez de Arenaza, Juan Carlos, E-31010 Pamplona (Navarra) (ES)
(72) Inventor: Chasco Pérez de Arenaza, Juan Carlos, E-31010 Pamplona (Navarra) (ES)
(74) Representative: Gislon, Gabriele
(86) International application number: PCT/ES2004/000247
(87) International publication number: WO 2004/109589

(57) **Abstract**

The invention relates to an intelligent laminated pressure surface. The inventive surface consists of: an elastic sheet which is designed to measure a determined external pressure and which is provided with numerous holes, said holes extending from the surface to the base thereof; and two sheets having inner conductive faces wich essentially cover both sides of said holes. According to the invention, the aforementioned pressure establishes a contact between the faces facing one another through the holes, thereby closing an electrical or optical conductor circuit. The invention can be used to locate determinded pressures, evaluate the frequencies and intervals thereof and measure and measure a pressure level or several pressure levels if more than one of the above-mentioned groups of sheets are disposed back-to-back. Moreover, the invention comprises an integrated conductor circuit and an antenna which alerts the user by means of an alarm or read device. The invention is particularly suitable for emdical applications, such as in the prevention of diabetes foot, decubitus ulcers, etc.

## Description

### Aim of the invention

This invention as explained in the title of this specification, relates to an improvement in the design and consequent use mainly for medical purposes of sheets with sensitive surfaces, which can receive information that can represent a risk for the wearer's skin.

### Background to the invention

The detailed description herein is mainly intended for medical application.

Patients can suffer from ailments which prevent them from noticing superficial pain (on their skin). For example, a small stone in their shoe. If a healthy person detects it, they easily solve the situation by removing the stone, however, anyone suffering from some specific ailments may not detect this foreign body and this can cause a wound that can worsen. This is typical and occurs frequently among those suffering from diabetes mellitus (4% of the population) who may suffer so-called diabetic neuropathy, with a high level of insensitivity. The resulting wound is the so-called diabetic ulcer, which has serious effects requiring long and expensive care, including long rest periods. In the US, almost 60,000 diabetic feet are amputated each year.

There are other cases such as the so-called decubitus ulcers, caused by an infrequently varied position (dementia, unconscious or coma patients...) in bed or when seated. Different areas of the anatomy suffer excess pressure, since the bones press on the skin and this in turn, is pressed by the piece of furniture on which the patient is resting: bed, stretcher, chair. These pressures can exceed a certain risk level and have a temporary frequency, which it is usually interesting to know. That is, it may be advantageous to know how long the patient remains in the same position; in the event of a long period of immobility, the medical team must know this, assess the risk level, and if necessary, change the patient's position.

To summarise, it is a question of obtaining superficial pressure data relative to the patient, data that the patient does not provide by being the actual patient. Surfaces with sensors are known which operate on the basis of distributing resistances, mainly piezoresistors and piezoelectric elements. In order to adequately distinguish the pressure applied in each point, they produce considerable output signals with their electronic components, conversors and other added elements. This therefore makes a foot insole uncomfortable. In order to solve this situation, the surface of this invention can discriminate each point with a hole, but the signals determining the risk contact can be much fewer, which considerably simplifies the conductive system.

There are also models based on elastic sheets with conductive areas, such as patent DE 4418775 A1 which registers manually adjustable pressure levels and is used in traumatology, and includes rigid elements next to the skin and is limited in discriminating small objects. Patents EP0286054A1 and DE8910258.4 U1, the latter being an insulating elastic sheet with rounded or oval holes and conductive buttons on the upper conductive surface which, vis-à-vis certain pressure, comes into contact with the lower conductive part thus activating an alarm, acting by area and discriminating areas with various holes. It measures a pressure level in each hole, and determines the risk pressures in pre-established areas. In diabetic feet, it does not accurately detect particularly small foreign risk objects, and neither does it assess the pressure frequencies, or claim a smart system or optical or radical conduction. The pressure surface model of this invention can overcome these drawbacks.

The pressure surface of this invention, via small output signals, can assess any risk pressure, by measuring various levels such as for example, 0, 1, 2, 3 or more, which, for example, in a liquid crystal reader, would be equivalent to four colours in each discriminated area, one colour for each level measured and facilitated by using a smart system.

### Disclosure of the invention

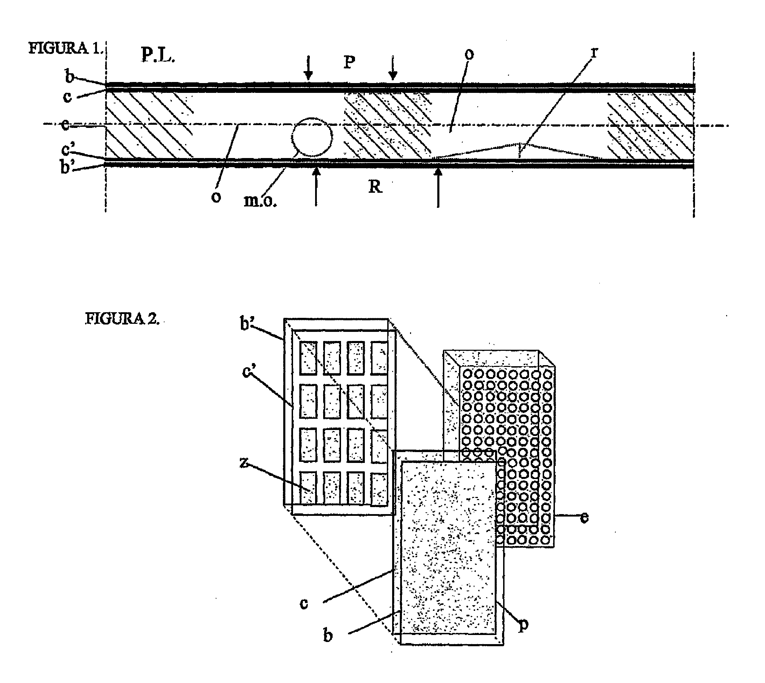

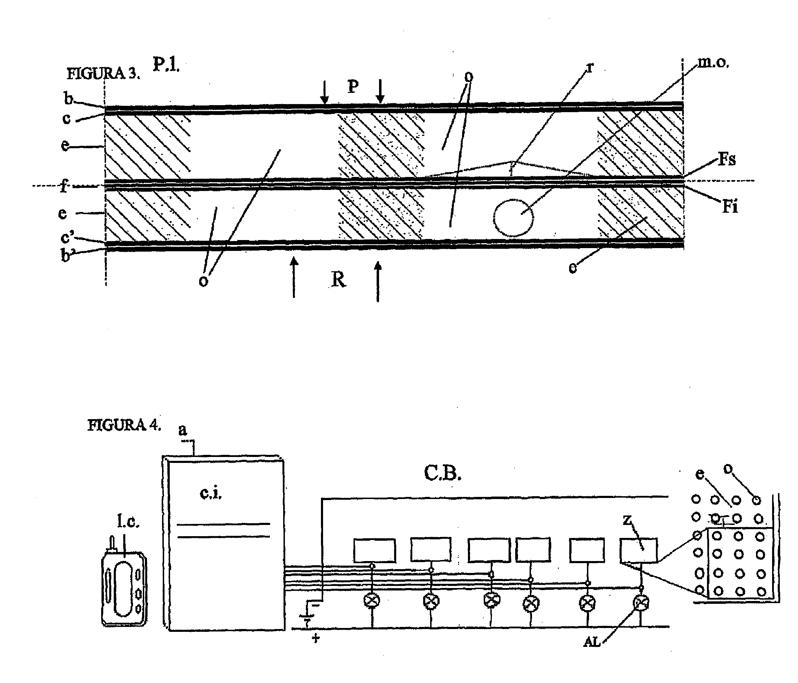

The device of this invention consists of a laminate pressure surface (fig. 1) comprising a non-conductive elastic sheet (e), with an abundance of holes (the shape and number of which is not claimed) which cross it from top to bottom; on both sides of said sheet there are two faces (c and c') or conductive sheets covering at least the holes thereof.

An external pressure (for example, produced by the object lodged between the shoe and the sole) that exceeds a certain measurement, brings into contact the conductive faces that face each other through the holes, whereby the user knows that there has been at least one point of contact somewhere in sheet (e).

The areas of the conductive faces facing the holes have a suitable relief r (figure 1) for facilitating said conductive contact (flat, pyramidal, conical, etc.); no particular shape is claimed; also optionally a mobile conductive object can be placed inside the holes (m.o. in Figure 1) to facilitate said contact.

The size and distribution of the holes makes it possible to detect any object whose pressure is sufficient to put the wearer's skin at risk.

The conductive faces can be insulated on the outside thereof (b and b') to stabilise the system, and the face (b) directly receiving the impact can be given a relief that facilitates small objects approaching the holes.

At least one of the conductive faces is distributed by areas (z, in Fig. 2), and the contacts with the holes reach these areas, and 1, 5, 10 or all the holes can reach them, according to the accuracy with which the user desires to know the location of the impacts (greater to less respectively). Each conductive area emits a signal (fig. 4).

### Brief description of the drawings

In order to improve the understanding of this specification description, several drawings are attached which represent, merely as an illustrative example, a practical embodiment of the smart laminate pressure surface (SPLI, in its abbreviated form in Spanish).
Figure 1. "P.L." section of smart laminate pressure surface, from the side, with insulating sheets (b) and (b'), conductive sheets (c) and (c'), elastic sheet (e), holes (o), relief (r) and an optional mobile object (e.g. a ball) which is also conductive (m.o).
Figure 2. Laminate pressure surface broken down into sheets: from top to bottom, in perspective (b), (c), (e) broken down towards the right, (c') and (b').
Figure 3. "P.L." section of smart laminate pressure surface, from the side, with insulating sheets (b), (f), (b'), conductive sheets (c), (Fs), (Fi), (c'), elastic sheets (e) and holes (o).
Figure 4. Basic circuit. "C.B." conductor, in this case, electrical: with generator, wiring, conducting areas (z) from which a signal is emitted, alarm devices (AL), reading device (I.c.), integrated circuit (c.i.), antenna (a) and as an example on the right of the drawing, holes (o) in an elastic sheet (e) that can receive a conductive area (z).
Figure 5. Smart laminate pressure surface, with its sheets broken down from top to bottom and in perspective: (b), (c), (e) broken down also towards the right, (Fs), (f), (Fi), (e) also broken down towards the right, (c') and (b'); (p) direct pressure receiver (objects, foot, etc.); (c') and (Fs) are distributed according to areas (z).
Figure 6. "C.T." section of smart laminate pressure surface, in the event of two possible foreign objects with different pressure: left, minimum pressure (m) and right, maximum pressure (M); top, pressure action "P", and bottom, pressure resistance "R", both with their indicator vectors, and the various sheets from top to bottom: (b), (c), (e), (Fs), (f), (Fi), (e), (c'), (b').

### Detailed description of a preferred embodiment

The device of this invention is a smart laminate pressure surface (SPLI in its abbreviated form in Spanish) comprising a non-conductive elastic sheet (e), with an abundance of holes (the shape and number of which are not claimed) that cross it from top to bottom; on both sides of said sheet there are two sheets, (c and c') the inner faces of which are conductive, and which mainly cover said holes.

A certain pressure (for example, as mentioned above, a shoe insole; the pressure produced by the object that lodged between the shoe and the bottom of the foot), brings into contact the conductive faces facing each other through the holes, therefore forming a conductive circuit.

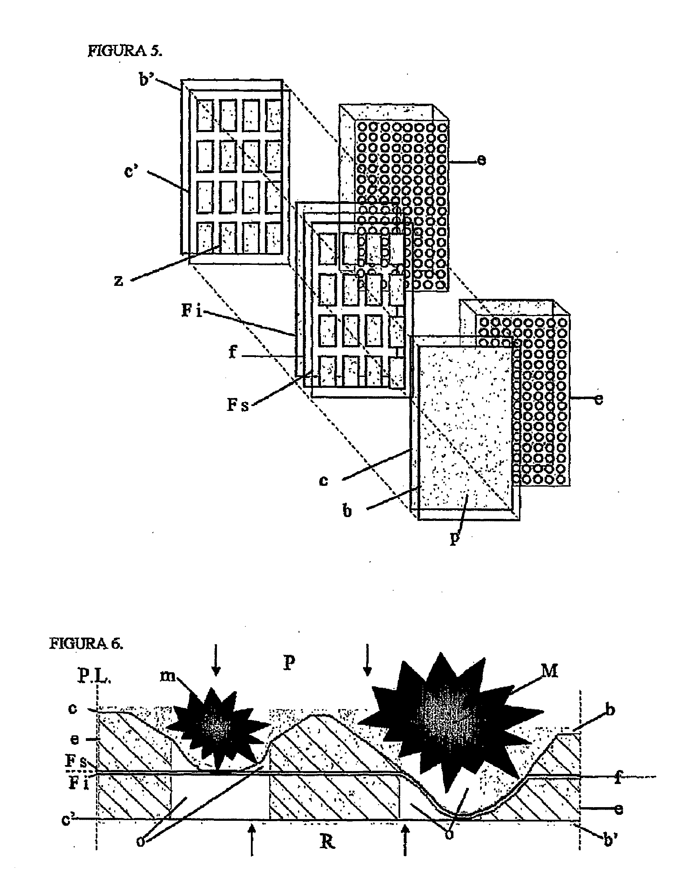

By means of these three sheets a pressure level can be measured, with each new level to be measured involving another laminate pressure group such as the one just described, backing onto it, (fig. 3) and with an adjustment of the corresponding physical-mechanical properties.

The areas of the conductive faces facing the holes have a suitable relief (r, fig. 1) for facilitating said conductive contact (flat, pyramidal, conical, etc.), which is not claimed; said contact is also facilitated by optionally installing a mobile object, for example, rounded: "m.o." in fig. 1, which goes inside the holes, and neither the shape or composition of said object is claimed.

The size and distribution of the holes makes it possible to notice any object whose pressure puts the wearer's skin at risk.

The face directly receiving the impacts (always "b", in the various drawings) has a relief that facilitates small objects approaching the holes.

The conductive faces are distributed in areas ("z" in figs. 2 and 4), and the contacts with the holes reach these areas, and 1, 5, 10 or all the holes can reach them, according to the accuracy with which the user desires to know the location of the impacts (respectively from greater to lesser pressure). Each conductive area emits a signal (fig. 4). The size and distribution of the areas on each conductive face are adjusted to the space to be measured. (Let's say the system is prepared to detect any small risk object, but it may be of interest that the location of the object is more or less accurate).

Fig. 6 shows on the left-hand side a "minimum contact" (m) with a foreign object, via the external pressure for example of a foot, which deforms the laminate pressure surface downwards, that allowing the inner face of sheet (c) to come into contact with the upper face (Fs) of f, both conductive faces, which the circuit detects. And the same figure 6 shows, on the right-hand side, an example of "maximum" contact which brings the lower face (Fi) of f into contact with the upper face (c') of both conductive elements, and the circuit also detects this contact. The smart laminate pressure surface (SPLI, in its abbreviated form in Spanish) indicates to the wearer that there are risk objects in his/her shoe and that he/she can remove them. As for the shape of the laminate pressure surface, this can be standard or made to measure. For example, insoles for various shoe sizes; surfaces of the size of a hospital bed sheet, or smaller surfaces, to monitor particular areas of the patient's bed.

The circuit comprises an accurate generator, either electrical (fig. 4), or optical or both combined, since the signals conducted may be of the three types.

The smart laminate pressure surface (SPLI, in its abbreviated form in Spanish) can also assess pressure or impact frequencies, with its varied measurements, and the interruption frequencies.

All the information is gathered (fig. 4) via the wiring and taken to alarm devices (AL) (that can be optical, acoustic, or combined optical and acoustical devices) or to reading devices (I.c) also in a smart integrated circuit (c.i.) which interprets them according to the program, and notifies the wearer. The signals can be conducted or radiated (a).

Finally, if it is desirable to determine the location of the pressure contacts, the two conductive faces are distributed according to areas, of varying size, with a signal being emitted from each area, although there may be several simultaneous contacts, and each area can comprise one, several or all of the holes.

If it is desirable to measure more than one pressure level, two or more pressure surfaces can be stacked, sandwich style, joining the faces of the laminate pressure surfaces that come into contact, and just considering the modifications made.

Its application is mainly medical: such as in the prevention of diabetic foot and decubitus ulcers. It is also intended for non-medical applications: where it is desirable to detect one or several pressures. Acting as a mechanism or with a servomechanism, to prevent a risk (for example in safety and footwear), for keyboard operation, and for diagnosing pressure distributions over a surface.

## Claims

1. Pressure surface of the type including elastic sheet and non-conducting holes, with two conducting faces backing onto both sides of said insulating sheet where a certain external pressure deforms it, enabling an electrical conducting contact between the conducting faces inside the holes, with a signal being emitted from a group of said holes. It is **characterised in that**: A) The relief of the conducting areas poking through the holes, or that of a mobile conducting object inside said holes, is suitable for enabling the conducting contact that is produced at one or several levels (upwards, downward or from side areas). B) In some devices the conducting contact is optical or a combination of both, no particular one is claimed. C) The relief of the face of the stabilising sheet (b) which receives the impacts directly from above, is suitable for improving the sensory contact of the small objects that can become lodged between the sole of the foot and the pressure surface. D) The addition of stabilising sheets (always optional), mainly in the upper and lower ends (b) and (b'). E) The presence of a microcomputer or smart circuit with its programs and properties. F) The conducting areas can have several holes or just one. G) The holes are distributed all over the risk areas, which vis-à-vis the sole of the foot, is all over said sole of the foot.

2. According to claim 1. The "sandwich" or stacking arrangement of pressure surfaces, which joins the elastic sheet and the holes, and enables the conducting faces to cover, look towards or intersect (f) the holes at the various levels.

3. According to claim 2. The localisation of the pressure: point, stripe and surface; the measurement of the frequencies and more than one pressure level.

4. According to claim 3. The conducting areas at the various levels adapt in size and distribution to the risk surfaces to be measured, with the conducting system achieving simplicity and maximum discrimination with many holes (as many as necessary) and minimum accurate output signals; no particular conducting system is claimed.

5. Surface like the described claims. With any contour (the material and the shape and other properties are not claimed).

6. According to the preceding claims the system is capable of detecting and discriminating any risk pressure, mainly those pressures generated by the body itself on the skin and by the objects becoming lodged between the skin and the pressure surface. No claim is made as to the particular relief or shape of the conducting faces poking through the holes or of the conducting mobile object lying inside said holes, or particular generators, reading or alarm devices, microprocessors or antennae.

Pressure surface of the type including elastic sheet and non-conducting holes, with two conducting faces backing onto both sides of said sheet: a certain external pressure deforms it, enabling an electrical conducting contact between the conducting faces inside the holes.
The surface in "sandwich" layers is **characterised in that** : A. It measures the pressure at one or several points or on a surface, more than one pressure level and automatically. B. Its various sheets stacked or joined at the various levels, have their physical-mechanical properties adjusted. C. The signals from the conducting areas comprising the contacts between one or several holes at the various levels are digitalised: an integrated or smart system that measures the frequency of the surface contacts, receives and sends the information by various means (electrical, optical, by cable or radio) and in various forms (light signal, acoustic signal, on the screen). These referenced forms and means of information are not claimed.
According to 1. The "sandwich" or "stacked" arrangement of more than one pressure surface, adjusting the physical-mechanical properties to various levels, (fig. 3), when external pressure applied, enables the contact between the conducting faces covering the holes at the different heights, according to the pressure intensity and favoured by a relief for said contact inside the holes. The relief of the conducting faces looking towards the holes, can be conical, pyramidal etc. No particular relief is claimed.
According to 1 and 2. Joining the sheets with holes in a "sandwich" arrangement, generates a sheet with different physical-mechanical properties according to the level or height and one single common hole at the various levels, with different conducting areas poking through said hole at the different levels. An external pressure enables the contact between said conducting areas, and this varies according to the pressure intensity. The conducting contact is electrical, optical or a combination of both.
According to 2 and 3. A mobile conducting device inside the hole, also enables the conducting contact between the various conducting areas that poke through the hole. No particular shape of said device is claimed.
According to the preceding claims. The sheet that receives the pressure impacts directly has a relief that favours small objects approaching the holes. Also the "sandwich" pressure surface includes stabilising sheets which serve mainly to insulate the system (b, b'). No particular stabilising sheet or relief thereof is claimed.
